(19) Europäisches Patentamt
European Patent Office
Office européen des brevets

(11) Publication number: 0 072 762
B1

(12) EUROPEAN PATENT SPECIFICATION

(45) Date of publication of patent specification: 11.09.85

(51) Int. Cl.[4]: C 07 C 87/26, A 61 K 31/13

(21) Application number: 82401539.0

(22) Date of filing: 16.08.82

# (54) Fluorinated diaminoalkene derivatives.

(30) Priority: 19.08.81 GB 8125360
19.08.81 GB 8125392

(43) Date of publication of application:
23.02.83 Bulletin 83/08

(45) Publication of the grant of the patent:
11.09.85 Bulletin 85/37

(84) Designated Contracting States:
AT BE CH DE FR IT LI LU NL SE

(56) References cited:
EP-A-0 025 370
EP-A-0 046 713
GB-A-2 003 876

Biochemical and Biophysical Research Comm.,
Vol. 67, No. 1, 1975, pages 392-395

(73) Proprietor: MERRELL TORAUDE ET COMPAGNIE
16 Rue d'Ankara
F-67084 Strasbourg (FR)

(72) Inventor: Danzin, Charles
18 rue Geiler
F-67000 Strasbourg (FR)
Inventor: Gerhart, Fritz
Rheinauerstrasse 14
D-7640 Kehl-Leutesheim (DE)
Inventor: van Dorsselaer, Viviane
9 rue du Hohneck
F-67100 Strasbourg (FR)

(74) Representative: Burford, Anthony Frederick et al
W.H. Beck, Greener & Co. 7 Stone Buildings
Lincoln's Inn
London WC2A 3SZ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 072 762 B1

**Description**

The invention relates to pharmaceutically useful fluorinated alkenylene diamine derivatives which *in vivo* are inhibitors of a decarboxylase enzyme involved in polyamine formation in organisms. The invention provides the compounds *per se*, pharmaceutical compositions comprising said compounds, methods of medical treatment using said compounds, and processes for preparing said compounds.

Background of the invention

The decarboxylation of ornithine to putrescine, a reaction catalyzed by the enzyme ornithine decarboxylase (ODC), is the first step in the biosynthesis of the polyamines known as spermidine and spermine. Spermidine is formed by the transfer of an activated aminopropyl moiety from S-adenosyl S-methyl homocysteamine to putrescine, while spermine is formed by the transfer of a second aminopropyl group to spermidine. S-Adenosyl S-methyl homocysteamine is formed by the decarboxylation of S-adenosylmethionine (SAM), a reaction catalyzed by the enzyme S-adenosylmethionine decarboxylase (SAM-DC).

The polyamines, which are found in animal tissues and microorganisms, are known to play an important role in cell growth and proliferation. The onset of cell growth and proliferation is associated with both a marked increase in ODC activity and an increase in the levels of putrescine and the polyamines. Although the exact mechanism of the role of the polyamines in cell growth and proliferation is not known, it appears thao the polyamines may facilitate macromolecular processes such as DNA, RNA, or protein synthesis. Polyamine levels are known to be high in embryonic tissue; in the testes, ventral prostrate, and thymus; in tumor tissue; in psoriatic skin lesions; and in other cells undergoing rapid growth or proliferation.

Since putrescine is the precursor of both spermidine and spermine, blockade of the conversion of ornithine to putrescine, such as by inhibition of ODC, should prevent new biosynthesis of these polyamines and, thus, provide beneficial physiological effects.

We have disclosed in U.K. Patent Specification No. 2001960A that *inter alia* compounds of the following Formula A are inhibitors of decarboxylase enzymes involved in polyamine formation:—

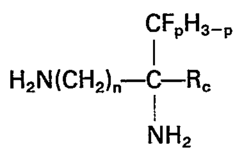

Formula A

wherein:—

$R_c$ represents $-COR_5$, where $R_5$ represents hydroxy or $C_1-C_8$ alkoxy;

*n* represents 3 or 4; and

*p* represents 1 or 2.

The compounds of Formula A in which *n* is 3 are disclosed as ornithine decarboxylase inhibitors and those in which *n* is 4 are disclosed as lysine decarboxylase inhibitors.

Further, we have disclosed in U.K. Patent Specification No. 2003876A that the analogues of said compounds of Formula A in which $R_c$ represents hydrogen are likewise decarboxylase enzyme inhibitors.

Summary of the invention

The compounds of the invention are represented by the following general Formula I:—

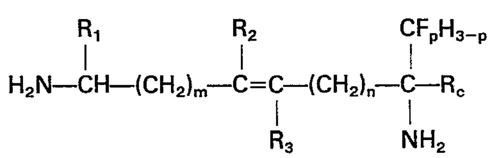

Formula I

wherein:—

$R_c$ represents hydrogen or $-COR_5$, where $R_5$ is as defined below;

$R_1$ represents hydrogen or $C_1-C_6$ alkyl;

one of $R_2$ and $R_3$ represents hydrogen and the other represents $C_1-C_6$ alkyl;

$R_5$ represents hydroxy or $C_1-C_8$ alkoxy;

*m* and *n* independently represent 0 or 1 but $m+n=0$ or 1; and

*p* represents 1 or 2.

Pharmaceutically acceptable salts and individual optical isomers of the compounds of general Formula I are also within the scope of the invention.

It is believed that the compounds of general Formula I are "substrate-induced irreversible inhibitors" of ornithine decarboxylase. Such inhibitors are also known in the art as "enzyme-activated irreversible inhibitors", "suicide enzyme inhibitors", "$K_{cat}$ inhibitors", or "mechanism-based inhibitors". In order for a

compound to be a substrate-induced irreversible enzyme inhibitor, the compound must be a substrate for the *target* enzyme, and the compound must contain a latent reactive group susceptible to being unmasked as the result of the normal catalytic action of the enzyme. The unmasking of the latent reactive group by the action of the enzyme generates a reactive function which alkylates a nucleophilic residue present at the active site of the enzyme. Thus, there is formed a covalent bond between the inhibitor and the enzyme at the active site resulting in irreversible inactivation of the enzyme. Such inhibitors are extremely specific since the inhibitor must be a substrate for the target enzyme and since biotransformation of the inhibitor by the target enzyme is required before the enzyme is inactivated. Although it is believed that the compounds of general Formula I generally exert their action by means of a substrate-induced mechanism, inhibition may occur by other mechanisms, such as by competitive inhibition.

The compounds of Formula I inhibit ornithine decarboxylase enzyme (ODC) *in vivo*, and produce a decrease in putrescine and spermidine concentrations in cells in which active biosynthesis is taking place. The compounds of Formula I, therefore, are useful in mammals for controlling undesirable cell growth or proliferation. The compounds of Formula I are useful pharmacological agents for treating those diseases or conditions that are known in the art to be characterized by ODC activity. In particular, the compounds are useful systemically for controlling the growth of tumor tissues in mammals, for treating benign prostatic hypertrophy, and for controlling the growth of pathogenic parasitic protozoa in infected domestic animals and humans.

The compounds of Formula I can also be employed to study the presence and physiological function of ODC inhibition in biological systems and its relationship to pathological processes.

The compounds of Formula I wherein $R_c$ is an ester group do not inhibit ODC *in vitro*. In order to produce inhibition of ODC *in vivo*, said compounds must undergo biotransformation to the compounds of Formula I wherein $R_c$ is hydrogen or carboxy, which compounds are inhibitors of ODC both *in vitro* and *in vivo*.

It will be recognised that the compounds of Formula I can be substituted at the carboxyl group, if present, and/or an amino group with any group known in the art to be capable of cleavage *in vivo* (enzymatically or chemically) to generate a free carboxylic and/or amino group. Compounds which contain such cleavable substituents and which, therefore, can be converted *in vivo* into a compound of Formula I will be equivalent to the compounds of Formula I for the purpose of this invention. Such derivatives can be prepared in manner known *per se* from the compounds of Formula I. A presently preferred derivative is N-glutamyl.

The ODC activity of the compounds can be determined *in vitro* by the method described by B. Metcalf *et al. J. Am. Chem. Soc., 100,* 2551 (1978). The ODC activity of the compounds of Formula I can be determined *in vivo* by the method of C. Danzin, *Biochemical Pharmacology, 28,* 627 (1979).

Detailed description of the invention

In the above general Formula I, $R_c$ represents hydrogen, carboxy (i.e. $R_5$ is hydroxy) or alkoxycarbonyl (i.e. $R_5$ is $C_1$—$C_8$ alkoxy).

In the above general Formula I, $R_1$, $R_2$ and $R_3$ independently represent hydrogen or $C_1$—$C_6$ alkyl, especially methyl except that one of $R_2$ and $R_3$ must be hydrogen and the other of $R_2$ and $R_3$ must be alkyl. Preferably $R_1$ is hydrogen and it is further preferred that $R_3$ is hydrogen.

References in this Specification, including the Claims, to an alkyl group or moiety mean a straight or branched chain alkyl group or moiety and, in the case of an alkyl group or moiety having structural isomers, includes all of those isomers and mixtures thereof unless a particular isomer is specified or clearly implied by the context.

Illustrative examples of straight or branched chain alkyl groups or moieties having 1 to 4 carbon atoms are methyl, ethyl, *n*-propyl, *iso*-propyl and *n*-butyl.

Illustrative examples of straight or branched chain alkyl groups or moieties having 1 to 6 carbon atoms are those specified above having 1 to 4 carbon atoms and *n*-pentyl, *neo*-pentyl, *n*-hexyl and *iso*-hexyl.

Illustrative examples of straight or branched chain alkyl groups or moieties having 1 to 8 carbon atoms are those specified above having 1 to 6 carbon atoms and *n*-heptyl, 5-methylhexyl and *n*-octyl.

It will be appreciated that when *m* and *n* are both 0 the compounds of the invention specified in Table I below are the alkyl-substituted fluorinated methyl dehydro analogues of the respective specified naturally occurring amino acid or diamine.

TABLE I

| Formula I | | | | |
|---|---|---|---|---|
| $R_1$ | $R_a$ | $R_b$ | $R_c$ | Analogue |
| H | H | H | $CO_2H$ | ornithine |
| H | H | H | H | putrescine |

In the above general Formula I, one of *m* and *n* is 0 and the other of *m* and *n* is 0 or 1. Further *p*

represents 1 or 2. It will be appreciated that when $p$ represents 1, the compounds of the invention are mono-fluoromethyl derivatives and that when $p$ represents 2 they are difluoromethyl derivatives. It presently is preferred that $m$ and $n$ are both 0 and independently that $p$ is 1.

As indicated in general Formula I, the compounds of the invention are in the trans, or entgegen, configuration. Trans isomers are indicated in the nomenclature used in this Specification, including the Claims, by the letter "E". The invention includes, of course, non-toxic mixtures of said isomer with its cis isomer.

Illustrative examples of pharmaceutically acceptable salts of the compounds of this invention include non-toxic acid addition salts formed with inorganic acids, such as hydrochloric, hydrobromic, sulfuric and phosphoric acid, or with organic acids, such as, organic carboxylic acids, for example salicylic, maleic, malonic, tartaric, citric and ascorbic acids, and organic sulfonic acids, for example methane sulfonic acid; and non-toxic salts formed with inorganic or organic bases, such as, hydroxides of alkali metals, for example, sodium, potassium and lithium, alkaline earth metals, for example, calcium and magnesium, light metals of Group III A, for example, aluminium, organic amines, such as, primary, secondary or tertiary amines, for example, cyclohexylamine, ethylamine, methylaminoethanol, ethanolamine and piperidine. The salts are prepared by conventional means.

In one preferred embodiment of the invention, there are provided compounds of the following general Formula IA:—

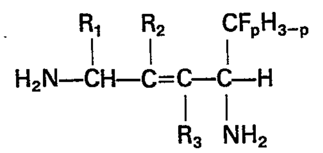

Formula IA

wherein $R_1$, $R_2$, $R_3$, and $p$ are defined in connection with Formula I; and pharmaceutically acceptable salts thereof.

In a preferred second embodiment of the invention, there are provided compounds of the following general Formula IB:—

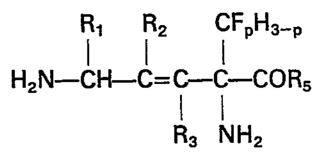

Formula IB

wherein $R_1$, $R_2$, $R_3$, $R_5$, and $p$ are as defined in connection with Formula I; and pharmaceutically acceptable salts thereof.

In a third embodiment of the invention, there are provided compounds of the following general Formula IC:—

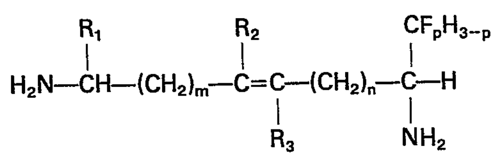

Formula IC

wherein $R_1$, $R_2$, $R_3$, *m, n, and p* are defined in connection with Formula I except that $m+n=1$; and pharmaceutically acceptable salts thereof.

In a fourth embodiment of the invention, there are provided compounds of the following general formula ID:

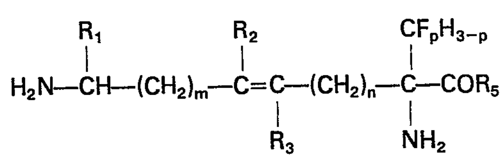

Formula ID

wherein $R_1$, $R_2$, $R_3$, $R_5$, $m$, $n$ and $p$ are as defined in connection with Formula I except that $m+n=1$; and pharmaceutically acceptable salts thereof.

It is especially preferred that in Formulae IA to ID both $R_1$ and $R_3$ are hydrogen.

Illustrative examples of compounds of the present invention are the following:—

1-fluoro-2,5-diamino-3-methyl-3-(E)-pentene;

1,1-difluoro-2,5-diamino-3-methyl-3-(E)-pentene;

2-fluoromethyl-2,5-diamino-3-methyl-3-(E)-penten-1-oic acid;

2-difluoromethyl-2,5-diamino-3-methyl-3-(E)-penten-1-oic acid;
ethyl 2-fluoromethyl-2,5-diamino-3-methyl-3-(E)-penten-1-oate;
ethyl 2-difluoromethyl-2,5-diamino-3-methyl-3-(E)-penten-1-oate;
1-fluoro-2,5-diamino-4-methyl-3-(E)-pentene;
1,1-difluoro-2,5-diamino-4-methyl-3-(E)-pentene;
2-fluoromethyl-2,5-diamino-4-methyl-3-(E)-penten-1-oic acid;
2-difluoromethyl-2,5-diamino-4-methyl-3-(E)-penten-1-oic acid;
ethyl 2-fluoromethyl-2,5-diamino-4-methyl-3-(E)-penten-1-oate;
ethyl 2-difluoromethyl-2,5-diamino-4-methyl-3-(E)-penten-1-oate;
1-fluoro-2,5-diamino-3-ethyl-3-(E)-pentene;
1,1-difluoro-2,5-diamino-4-ethyl-3-(E)-pentene;
2-fluoromethyl-2,5-diamino-4-ethyl-3-(E)-penten-1-oic acid;
2-difluoromethyl-2,5-diamino-3-ethyl-3-(E)-penten-1-oic acid;
1-fluoro-2,5-diamino-4-propyl-3-(E)-pentene;
1,1-difluoro-2,5-diamino-3-hexyl-3-(E)-pentene;
2-fluoromethyl-2,5-diamino-4-hexyl-3-(E)-penten-1-oic acid;
2-difluoromethyl-2,5-diamino-3-butyl-3-(E)-penten-1-oic acid;
1-fluoro-2,5-diamino-4-methyl-3-(E)-hexene;
1,1-difluoro-2,5-diamino-3-methyl-3-(E)-hexene;
1-fluoro-2,5-diamino-4-methyl-3-(E)-heptene;
1,1-difluoro-2,5-diamino-4-methyl-3-(E)-heptene;
1-fluoro-2,6-diamino-3-methyl-3-(E)-hexene;
1,1-difluoro-2,6-diamino-3-methyl-3-(E)-hexene;
2-fluoromethyl-2,6-diamino-3-methyl-3-(E)-hexen-1-oic acid;
2-difluoromethyl-2,6-diamino-3-methyl-3-(E)-hexen-1-oic acid;
1-fluoro-2,6-diamino-4-methyl-3-(E)-hexene;
1,1-difluoro-2,6-diamino-4-methyl-3-(E)-hexene;
2-fluoromethyl-2,6-diamino-4-methyl-3-(E)-hexen-1-oic acid;
2-difluoromethyl-2,6-diamino-4-methyl-3-(E)-hexen-1-oic acid;
1-fluoro-2,6-diamino-5-methyl-4-(E)-hexene;
1,1-difluoro-2,6-diamino-5-methyl-4-(E)-hexene;
2-fluoromethyl-2,6-diamino-5-methyl-4-(E)-hexen-1-oic acid;
2-difluoromethyl-2,6-diamino-5-methyl-4-(E)-hexen-1-oic acid;
1-fluoro-2,6-diamino-4-methyl-4-(E)-hexene;
1,1-difluoro-2,6-diamino-4-methyl-4-(E)-hexene;
2-fluoromethyl-2,6-diamino-4-methyl-4-(E)-hexen-1-oic acid;
2-difluoromethyl-2,6-diamino-4-methyl-4-(E)-hexen-1-oic acid;
1-fluoro-2,6-diamino-3-ethyl-3-(E)-hexene;
1,1-difluoro-2,6-diamino-4-ethyl-4-(E)-hexene;
2-fluoromethyl-2,6-diamino-5-ethyl-3-(E)-hexen-1-oic acid;
2-difluoromethyl-2,6-diamino-4-ethyl-3-(E)-hexen-1-oic acid;
1-fluoro-2,6-diamino-4-propyl-3-(E)-hexene;
1,1-difluoro-2,6-diamino-5-hexyl-4-(E)-hexene;
2-fluoromethyl-2,6-diamino-4-hexyl-4-(E)-hexen-1-oic acid;
2-difluoromethyl-2,6-diamino-3-butyl-3-(E)-hexen-1-oic acid;
1-fluoro-2,6-diamino-4-methyl-3-(E)-heptene;
1,1-difluoro-2,6-diamino-4-methyl-4-(E)-heptene;
1-fluoro-2,6-diamino-5-methyl-4-(E)-octene;
1,1-difluoro-2,6-diamino-4-methyl-3-(E)-octene.

As used herein, the term "tumor tissue" means both benign and malignant tumors or neoplasms, and includes leukemias, lymphomas, melanomas, and sarcomas. The term "controlling the growth of tumor tissue; as used herein means slowing, interrupting, arresting, or stopping the growth of a rapidly proliferating tumor in a warm blooded animal. It should be understood that the administration of a compound of the Formula I does not provide a "cure" for the tumor in the sense that the tumor tissue is destroyed or totally eliminated from the animal being treated.

For controlling the growth of tumor tissues, a compound of Formula I can be administered to the patient in conjunction with other therapeutic methods or in combination with cytotoxic drugs known in the art to be useful for cancer chemotherapy. For example, a compound of Formula I can be administered in conjunction with surgical excision of the tumor or with radiation therapy, hormonal treatment, immunotherapy, or local heat therapy. Moreover, in a preferred manner, a compound of Formula I can be administered to a patient in combination with a chemical cytotoxic agent known in the art to be useful for tumor chemotherapy. When such combination therapy is employed for the treatment of a tumor, the cancer chemotherapeutic agent may be administered at a dosage known in the art to be effective for treating the tumor. However, a compound of Formula I may produce an additive or synergistic effect with a chemotherapeutic agent against a particular tumor. Thus, when such combination antitumor therapy is

used, the dosage of the chemotherapeutic agent administered may be less than that administered when the agent is used alone. In combination with a compound of Formula I, the chemotherapeutic agent may, therefore, be administered at a lower dosage level or at less frequent intervals as compared to the chemotherapeutic agent when used alone.

In combination with a compound of Formula I, any cancer chemotherapeutic agent may be employed. Drugs commonly used for cancer chemotherapy are described in *The Medical Letter*, Vol. 22, No. 24 (Issue 571), November 28, 1980, Published by the Medical Letter, Inc., New Rochelle, N.Y., 10801. Illustrative examples of cytotoxic chemotherapeutic agents are cyclophosphamide, methotrexate, prednisone, 6-mercaptopurine, procarbozine, daunorubicin, vincristine, vindesine, vinblastine, chlorambucil, cytosine arabinoside, 6-thioguanine, thio TEPA, 5-fluorouracil, 5-fluoro-2-deoxyuridine, 5-azacytidine, nitrogen mustard, 1,3-bis(2-chloroethyl)-1-nitrosourea (BCNU), 1-(2-chloroethyl)-3-cyclohexyl-1-nitrosourea (CCNU), busulfan, adriamycin, bleomycin, cycloleucine or methylglyoxal bis(guanylhydrazone) (MGBG). Other cancer chemotherapeutic agents will be apparent to those skilled in the art.

The effect of the compounds of Formula I for the control of the growth rate of rapidly proliferating tumor tissue can be assessed in standard animal tumor models after oral or parenteral administration. For example, the antitumor effects can be demonstrated in the following models: (a) L1210 leukemia in mice, (b) EMT 6 tumor in Balb/C mice, (c) 7,12-dimethylbenzanthracene-induced (DMBA-induced) mammary tumors in rats, or (d) Morris 7288 C or 5123 hepatoma in Buffalo rats. In addition, the antitumor effects of the compounds in combination with chemotherapeutic agents can be demonstrated in animal models.

When, in the treatment of a malignant neoplastic disease, a compound of Formula I is administered in combination with a chemotherapeutic agent, the therapeutic effect of the chemotherapeutic agent may be potentiated in that the remission produced by the chemotherapeutic agent may be enhanced and regrowth of the tumor tissue may be slowed or prevented. Use of such combination therapy therefor allows smaller doses or fewer individual doses of the chemotherapeutic agent to be employed. Thus, the detrimental and/or debilitating side effects of the chemotherapeutic agent are minimized while, at the same time, the antitumor effects are enhanced. The term "combination therapy" contemplates the administration of a compound of Formula I immediately prior to the beginning of chemotherapy, concomitantly with chemotherapy, or during the period of time immediately following cessation or discontinuance of chemotherapy.

When chemotherapy results in remission of the tumor and all tumor cells are not destroyed, regrowth of the tumor may be prevented or slowed indefinitely by continued treatment with a compound of Formula I. Thus, a compound of Formula I can be administered to stop or slow the growth of the tumor during the periods when chemotherapy using a cytotoxic agent may be temporarily discontinued.

A preferred cytotoxic agent for combination therapy with a compound of Formula I is methylglyoxal bis(guanylhydrazone), herein referred to as MGBG, which is also an inhibitor of S-adenosyl methionine decarboxylase. The activity of MGBG is a chemotherapeutic agent in the treatment of neoplastic diseases is well documented. For example, W. A. Knight *et al. Cancer Treat. Rep., 43,* 1933, (1979) have reported that a dose of MGBG administered intravenously once or twice a week to patients in the advanced stages of carcinoma of the bladder, esophagus, lung, pancreas, colon, kidney, breast and prostate, oat cell carcinoma, adenocarcinoma, lymphoma, hepatoma, melanoma, leukemia, or Edwing's sarcoma produced measurable regression of the tumor in many of the patients treated and complete disappearance of the diseases in two of the 65 treated patients.

The amount of MGBG to be administered may be the same as the amount known in the art to be effective for tumor therapy. Effective and non-toxic dosages are determined by the physician in each case, taking into account the condition of the individual patient. For example, a dosage of 250—500 mg per meter$^2$ of body surface area may be infused once or twice weekly in 100 ml of aqueous 5% dextrose solution over a 30 min period. Combination therapy with a compound of Formula I improves the response of the tumor tissue to the cytotoxic effect of MGBG and permits the use of a smaller individual dose of MGBG and a shorter course of treatment than would be required with the use of MGBG alone.

Suitable dosages of the compounds of Formula I for use in combination therapy with MGBG or other cancer chemotherapeutic agents can be any amount effective in inhibiting polyamine biosynthesis sufficiently to control the tumor growth rate or to achieve a heightened response to the cytotoxic agent administered in conjunction therewith.

The term "controlling the growth of pathogenic parasitic protozoa", as used herein, means slowing, interrupting, arresting, or stopping the replication of the protozoa in an infected host. The compounds of Formula I are particularly useful against *T.b. brucei* (which causes trypanosomiasis in cattle). *T.b. rhodesiense*, (which causes human sleeping sickness), the coccidia, for example, *Eimeria tenella* (which causes intestinal coccidiosis in fowl (e.g. chickens, turkeys, and ducks)) and the exoerythrocytic form of *plasmodia*, for example, *plasmodium falciparum* (which causes human malaria).

The antiprotazoal activity of the compounds of Formula I can be demonstrated *in vivo* or *in vitro* in standard microbiological test procedures. For example, the activity of the compounds against *T.b. brucei*, and *T.b. rhodesiense* can be determined in infected mice by administering the test compound *ad lib* daily (3 to 15 days post infection) as a solution in the drinking water. Activity is indicated by an increase in survival time (as compared to untreated controls) or by the absence of parasites in the blood. The activity of the compounds against the coccidia can be determined in infected chickens, for example those infected with *E.*

*tenella* by administering the test compound daily *ad lib* (from one day pre infection to five days post infection) as a solution in the drinking water. The cecal lesions are evaluated by a standard lesion scoring procedure. (See Reid. *Am. J. Vet Res., 30,* 447 (1969) and *Avian Coccidiosis*, P. Long, Editor, British Poultry Science, Ltd., Edinburgh). The activity of the compounds against malaria (*p. falciparum*) can be determined by a standard *in vitro* plate culture test (See. K. Rieckmann et al, *Lancet, 1,* 22 (1978)). Antimalarial activity can also be determined in special strains of mice infected with the exoerythrocitic form of *p. berghei*. In this test, the compound is administered *ad lib* in drinking water starting two days preinfection and continuing 28 days post-infection. Activity is measured by a significant decrease in deaths as compared to controls or by a significant increase in survival time.

The compounds of Formula I wherein $R_c$ is —$COR_5$ are also capable of interrupting embryogenesis in female mammals when administered systematically. Thus, the compounds are useful as contragestational agents in female mammals when it is desired to terminate early pregnancy. The contragestational activity of the compounds can be demonstrated in mice by the method of J. Fozard, *European Journal of Pharmacology, 65,* 379 (180). In general, an effective daily dose of the compounds of Formula I, wherein $R_c$ is —$COR_5$, for terminating pregnancy in warm-blooded mammals is from 10 mg/kg to 1 g/kg, preferably 10 to 100 mg/kg, administered after fertilisation during the period between Standard Stages 8—16 of gestation as defined by E. Wischi (see Tables 26—27, pages 82—92, *Biology Data Book*, Altman and Dittmer, Editors, Published by the Federation of American Societies for Experimental Biology, Washington, D.C., 1964). The period of treatment will vary with the species. In humans, the period of treatment will extend from the 6th—7th day of gestation to the 27th day.

The compounds of this invention can be administered in various manners to achieve the desired effect. The compounds can be administered alone or in the form of pharmaceutical preparations either orally or parenterally, for example, subcutaneously, intravenously or interperitoneally. The amount of novel compound administered will vary and can be any effective amount. Depending upon the patient, the condition being treated and the mode of administration, the effective dosage of the compound administered may vary from about 5 mg/kg to about 100 mg/kg, of body weight of the patient per day. Unit doses of these compounds can contain, for example, from about 10 mg to 300 mg of the compounds and may be administered, for example, from 1 to 4 times daily.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

In the composition aspect of the invention there are provided pharmaceutical formulations in which form the active compounds of the invention will normally be utilized. Such formulations are prepared in a manner well known *per se* in the pharmaceutical art and usually comprise at least one active compound of the invention in admixture or otherwise in association with a pharmaceutically acceptable carrier or diluent therefor. For making these formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable carriers or diluents are well known *per se*.

The formulations of the invention may be adapted for enteral or parenteral use and may be administered to the patient in the form of tablets, capsules, suppositories, solutions, suspensions or the like.

In the specific examples included hereinbelow illustrative examples of suitable pharmaceutical formulations are described.

Methods of preparing the compounds of Formula I will now be described. If in any of the reaction steps described an amino group of a reactant would be involved in an unwanted reaction under the relevant reaction conditions, the amino group will be protected in manner known *per se* by introduction of an appropriate protecting group. The protecting group will be chosen having regard to the nature of the relevant reaction and ease of removal to free the amino group. The protecting group can be selected from, for example, acyl, for example, lower alkanoyl, e.g. acetyl, propionyl, trifluoroacetyl, and the like; aroyl, e.g. benzoyl, toluoyl and the like; lower alkoxycarbonyl, for example methoxycarbonyl, ethoxycarbonyl, *tert*-butoxycarbonyl and the like; carbobenzoxy, benzenesulfonyl and tosyl. Both amino hydrogen atoms can be substituted by a single protecting group such as, for example phthaloyl. The protecting groups are introduced in manner known *per se* by, for example, reaction of the amine with a lower alkanoyl or aroyl chloride, anhydride, sulfonylchloride, *tert*-butoxycarbonyloxyimino-2-phenyl-acetonitrile (BOC-ON), or di-tert-butyl dicarbonate ($(BOC)_2O$).

Removal of the protecting group after the required reaction has been completed can be carried out in manner known *per se* for the relevant protecting group. Usually, said removal will be by hydrolytic cleavage using a strong organic or mineral acid such as, for example, trifluoroacetic acid, hydrochloric acid and the like acids; or by hydrogen chloride gas under anhydrous conditions. The use of conditions which will react with the olefinic double bond or of reactants, such as hydrobromic acid, which will react with the olefinic double bond must be avoided. Solvents used will be chosen dependent upon the conditions of

protecting group removal. For example, ethers such as, for example, diethylether can be used for cleavage using hydrogen chloride gas.

The compounds of Formula I can be prepared in manner known *per se* from the corresponding compound of the following general Formula II:—

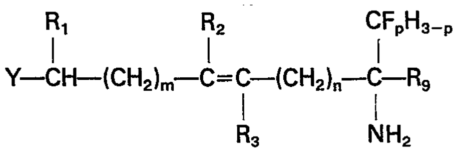

Formula II

wherein:

$R_1$, $R_2$, $R_3$, *m*, *n*, and *p* are defined in connection with Formula I;

$R_9$ represents hydrogen, cyano or $C_2$—$C_9$ alkoxycarbonyl; and

Y represents a leaving group such as hydroxy bromine, chlorine, iodine, tosyloxy (i.e. toluene-p-sulfonyloxy) or mesyloxy (i.e. methanesulfonyloxy).

The reaction can proceed via the corresponding phthalimido derivative, as described below.

The amino group in the compound of Formula II will be protected in manner known *per se* during the reaction by a suitable subsequently removable protecting group or groups. The protecting group preferably is phthaloyl. When proceeding via the phthalimido derivative when *p* is 1, it is necessary to use a protecting group which does not leave any hydrogen atom on the amino group in order to obtain the desired compound of Formula I. Usually, the protecting group will be selected so that it is removed during the final step in the conversion of the compound of Formula II into the corresponding compound of Formula I.

The amino-protected derivative of a compound of Formula II with an appropriate leaving group can be treated with an alkali metal phthalimide, especially sodium or potassium phthalimide, in a polar organic solvent, such as for example, dimethylformamide, dimethylsulfoxide or hexamethylphosphoric triamide, to form the corresponding phthalimido derivative. Any of the leaving groups Y exemplified above except hydroxy is appropriate for this reaction. Conveniently one to three equivalents of the phthalimide salt are used per equivalent of compound of Formula II at a temperature of 25° to 100°C for a period of 0.5 to 3 hours.

When Y is hydroxy, the amino-protected derivative of a compound of Formula II can be converted into the phthalimido derivative by reaction with phthalimide in the presence of a trialkyl- or triaryl-phosphine and diethylazodicarboxylate in an anhydrous aprotic solvent. Usually 1 to 3 equivalents each of phthalimide, the phosphine and diethyldiazodicarboxylate will be used per equivalent of alcohol reactant at a temperature of 10°C to 100°C for a period of 18 to 24 hours.

When $R_9$ is hydrogen or alkoxycarbonyl, the phthalimido derivative can be converted into the required compound of Formula I by heating with a reactant such as hydrazine or methylamine in a polar organic solvent such as, for example, an alkanol, preferably ethanol. Preferably hydrazine hydrate is used in an amount of about 2 equivalents per equivalent of phthalimido derivative. Suitably, the conversion is performed at 50° to 100°C, preferably under reflux conditions, for a period of 3 to 24 hours.

The phthalimido derivative of Formula II also can be converted into the required compound of Formula I by heating with a strong mineral acid such as hydrochloric acid or sulfuric acid. Said heating also hydrolyses any cyano group represented by $R_9$ to a carboxy group. Preferably a mixture of hydrochloric and acetic acid is used at a temperature of about 95°C for about 24 hours. Acids, such as hydrobromic acid, which are reactive towards olefinic double bonds cannot be used.

Compounds of Formula II above in which *m* is 0, $R_1$ is hydrogen and Y is bromine or iodine suitably can be obtained by boron tribromide or trialkylsilyliodide cleavage in manner known *per se* of an allylic compound of the following general Formula III:—

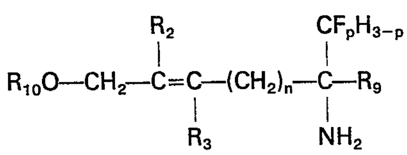

Formula III

wherein:

$R_2$, $R_3$, $R_9$, and *p* are as defined in connection with Formula II;

$R_{10}$ represents $C_1$—$C_4$ alkyl, preferably methyl; and

*n* is 0 or 1.

Compounds of Formula II in which *m* is 0, $R_1$ is hydrogen and Y is hydroxy also can be obtained from the corresponding compounds of Formula II in which Y is halogen by treatment with sodium acetate and acetic acid and subsequent reduction with, for example lithium aluminium hydride, of the resultant acetate. When a compound of Formula II in which $R_1$ is $C_1$—$C_6$ alkyl and Y is hydroxy is required, a compound of Formula II obtained by said reduction is oxidized with, for example, dimethylsulfoxide in the presence of

oxalyl chloride and triethylamine at about −78°C and the resultant aldehyde reacted with, for example, the appropriate alkyl lithium.

Compounds of Formula III in which $R_9$ represents cyano can be obtained from the corresponding compounds of the following general Formula IV by treatment with an alkali metal or ammonium cyanide, such as, for example, sodium cyanide in water in the presence of a water soluble ammonium salt of a strong acid, especially ammonium chloride.

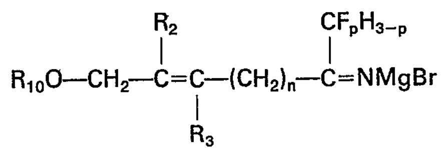

Formula IV

wherein:

$R_2$, $R_3$, $R_{10}$, *n* and *p* are as defined in connection with Formula III.

Compounds of Formula III in which $R_9$ represents hydrogen, can be obtained from the corresponding compound of Formula IV by reduction with a reducing agent, such as a borohydride, which selectively reduces the imino group.

Compounds of Formula III in which $R_9$ represents alkoxycarbonyl can be obtained by hydrolysis of the corresponding compound of Formula III in which $R_9$ represents cyano in the presence of an acid, such as hydrochloric acid, and the corresponding alcohol.

Compounds of Formula IV can be obtained by treatment of the corresponding Grignard reactant of the following general Formula V with the corresponding fluorinated acetonitrile of the following general Formula VI:—

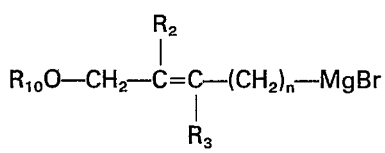

Formula V

wherein $R_2$, $R_3$, $R_{10}$ and *n* are as defined in connection with Formula IV;

$$CF_pH_{3-p}\text{—}CN \qquad \text{Formula VI}$$

wherein *p* represents 1 or 2.

The Grignard reactants of Formula V can be prepared in manner known *per se* from, for example, the corresponding bromides of the following general Formula VII and magnesium turnings in an appropriate solvent for Grignard type reactions.

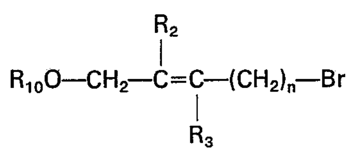

Formula VII

wherein $R_2$, $R_3$, $R_{10}$ and *n* are as defined in connection with Formula V.

The bromides of Formula VII are known or can be prepared by analogous processes for obtaining said known compounds.

In the case of compounds of Formula II in which *m* is 0, $R_1$ is hydrogen, $R_2$ is methyl and $R_9$ is hydrogen, the compounds of said formula can be prepared by allylic halogenation of a compound of the following Formula VIII

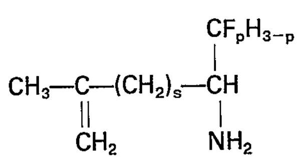

Formula VIII

wherein *s* is 1 or 2 and *p* is 1 or 2.

Conveniently, the halogenation can be carried out by the Wohl-Ziegler Reaction in which the compound of Formula VIII is treated with an N-haloamide, preferably an N-bromosuccinimide, usually in the presence of a free-radical initiator such as a peroxide or labile azo compound and under light irradiation.

The allylic halogenation of the compound of Formula VIII yields a mixture of the said compound of Formula II and the structural isomer of the following general Formula B:

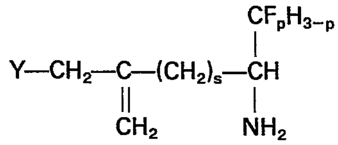

Formula B

wherein:

Y represents halogen; and

*p* and *s* are as defined in connection with Formula VIII.

These compounds can be separated in manner known *per se* but usually the mixture will be converted via the corresponding phthalimido derivative into a mixture of the corresponding diamines, which can then be separated by column chromatography of their di-BOC derivatives in the manner described below in connection with separation of acids of Formula I and Formula C.

Compounds of Formula VIII can be obtained by reducing with, for example, a borohydride the cyano compound obtained by treatment of a compound of the following Formula IX with an alkali metal or ammonium cyanide, such as, for example, sodium cyanide in water in the presence of a water soluble ammonium salt of a strong acid, especially ammonium chloride.

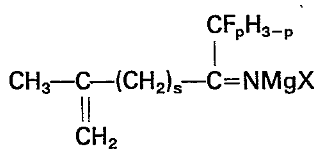

Formula IX

wherein *p* and *s* are as defined in connection with Formula VIII; and

X represents bromine, chlorine or iodine.

Compounds of Formula IX can be obtained by treatment of the corresponding Grignard reactant of the following general Formula X with the corresponding fluorinated acetonitrile of the following general Formula XI.

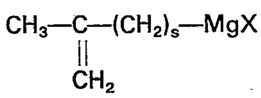

Formula X

wherein X and *s* are as defined in connection with Formula IX.

$$CF_pH_{3-p}—CN$$ Formula XI

wherein *p* is 1 or 2.

The Grignard reactants of Formula X can be prepared in manner known *per se* from, for example, the corresponding halides and magnesium turnings.

Compounds of Formula I in which *m* is 0, $R_2$ is methyl and $R_c$ is carboxy also can be obtained by acid hydrolysis of the di-phthalimido derivative of a compound of the following Formula XII.

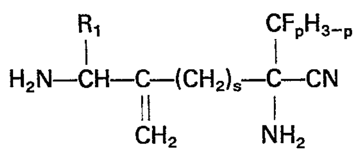

Formula XII

wherein $R_1$ and *p* are as defined in connection with Formula I; and *s* is 1 or 2.

Said hydrolysis yields a mixture of the said compound of Formula I with a compound of the following Formula C:—

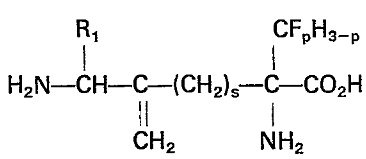

Formula C

wherein:

$R_1$, *p* and *s* are as defined in connection with Formula XII.

The said mixture compounds of Formula I and Formula C can be separated in manner known *per se* after derivatisation of the amino and carboxylic functions, for example first protecting both amino groups by treatment with *tert*-butoxycarbonyloxyimino-2-phenyl-acetonitrile (BOC-ON) and then forming the methyl ester by treatment with diazomethane, and separation of the di-BOC methyl esters in manner known *per se* by column chromatography. Subsequently, the separated derivatives can be treated in manner known *per se* to free the amino groups and/or the carboxy group. In connection with the derivatisation, it has been found that if the ester is formed without first protecting the amino groups, a cyclic product is obtained.

Compounds of Formula II in which *m* is 1, $R_1$ is hydrogen, $R_9$ represents hydrogen or cyano and Y represents hydroxy can be obtained by tribromide or trialkylsilyliodide cleavage in manner known *per se* of an amino-protected derivative of a compound of the following general Formula XIII to form the corresponding hydroxy compound.

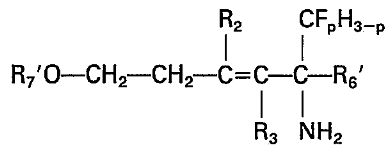

Formula XIII

wherein:

*p* is 1 or 2;

$R_6'$ represents hydrogen or cyano; and

$R_7'$ represents methyl or benzyl.

The reactant of Formula XIII can be prepared in analogous manner to that described above for the preparation of compounds of Formula III but commencing with bromination of the corresponding methyloxyalkane or benzyloxyalkane.

When compound of Formula II in which *m* is 1, $R_1$ is $C_1$—$C_6$ alkyl and Y is hydroxy is required, it can be prepared from the analogous compound of Formula II in which $R_1$ is hydrogen by oxidation and subsequent alkylation as described above in the case where *m* is 0.

It will be appreciated that the order of some of the reaction steps in the process routes described above can be changed.

The esters of Formula I wherein $R_c$ is alkoxycarbonyl can be obtained in manner known *per se* from the corresponding acids of Formula I wherein $R_c$ is carboxy by esterification with the corresponding alcohol or by conversion of the terminal-amine protected acid into the corresponding acid chloride and alcoholysis of said acid chloride with the corresponding alcohol.

When necessary in the preparation of compounds of Formula I separation of cis/trans isomers or intermediates or final products can be carried out by chromatographic techniques.

The compounds of Formula I contain at least one asymmetrical carbon atom and therefore exist as stereo-isomers. Methods of separating the stereoisomers of a particular compound will be apparent to those skilled in the art. For example, when $R_1$ is hydrogen, the individual optical isomers of the compounds of Formula I may be separated in manner known *per se* using optically active acids or bases. In particular, the amino group distal to the fluorinated methyl group can be protected using a ($C_2$—$C_5$ alkoxycarbonyl) phthalimide in a solvent such as, for example tetrahydrofuran, diethyl ether or $C_1$—$C_4$ alkanol, e.g. as methanol or ethanol. The protected amine derivative is then resolved using a chiral acid. The resolved phthalimido compound is then deprotected using, for example, hydrazine or methylamine to remove the phthalimide group followed if required by acid or base hydrolysis to cleave the ester product to obtain the corresponding acid. The thus resolved acids, esters and amines may be employed to prepare the individual isomers of other compounds of the invention in the manner described hereinabove.

The compounds produced by the foregoing processes may be isolated either *per se* or as acid addition salts thereof.

The acid addition salts are preferably the pharmaceutically acceptable, non-toxic addition salts with suitable acids such as those previously referred to in this Specification. Apart from pharmaceutically acceptable acid addition salts, other salts are also included within the scope of acid addition salts, such as for example, those with picric or oxalic acid; they may serve as intermediates in the purification of the compounds or in the preparation of other, for example, pharmaceutically acceptable, acid additions salts, or are useful for identification or characterisation of the bases.

A resulting acid addition salt may be converted into the free compound according to known methods, for example, by treating it with an alkali or alkaline earth metal hydroxide or alkoxide; with an alkali metal or an alkaline earth metal carbonate or hydrogen carbonate; with trialkylamine; or with an anion exchange resin.

A resulting acid addition salt may also be converted into another acid addition salt according to known methods; for example, a salt with a inorganic acid may be treated with a sodium, barium or silver salt of an acid in a suitable diluent, in which a resulting inorganic salt is insoluble and is thus removed from the reaction medium. An acid addition salt may also be converted into another acid addition salt by treatment with an anion exchange preparation.

The invention is illustrated by the following non-limiting Examples. All NMR measurements are given on the delta scale (i.e. tetramethylsilane=0).

Example 1
Preparation of 1-fluoro-2,5-diamino-4-methyl-3-(E)-pentene, dihydrochloride
A) 1-Fluoro-2-amino-4-methyl-4-pentene

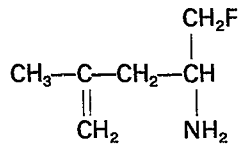

Under an atmosphere of nitrogen, methallylmagnesium chloride is prepared from 97.2 g (4 moles) of magnesium turnings, methallyl chloride (90.6 g, 1 mole) and dry tetrahydrofuran (900 mL). The Grignard solution is separated from the excess of magnesium, cooled to −40°C and fluoroacetonitrile (56 g, 950 mmoles) in dry tetrahydrofuran (200 mL) is added, dropwise, during about 1 hour. The reaction mixture is kept at −40°C for an additional 30 minutes, and then poured into a stirred mixture of methanol (2 L), water (50 mL) and sodium borohydride (39 g) cooled at −40°C. After stirring for 1 hour at −30°C, the temperature is allowed to rise to 0°C during 1 hour. After acidification with 6 N hydrochloric acid (about 500 mL) and evaporation, the residue is dissolved in water (about 2 L), and the solution is extracted 3 times with ether to remove non-basic by-products. The solution is made alkaline with 6 N sodium hydroxide and extracted 3 times with diethyl ether. The organic layer is dried over sodium sulfate and evaporation of the solvent affords 52.5 g of a colored oil (45%).

NMR ($CDCl_3$): 1.67 (2H, s, —$NH_2$), 1.77 (3H, s), 2.10 (2H, m), 3.30 (1H, m), 4.33 (2H, d of m, $J_{H-F}$=48 Hz), 4.87 (2H, m).

(B) 1-Fluoro-2-phthalimido-4-methyl-4-pentene

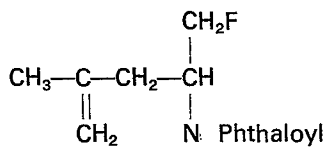

A mixture of 1-fluoro-2-amino-4-methyl-4-pentene (52.5 g, 450 mmoles) prepared as in step A above, N-carbethoxyphthalimide (98.55 g, 450 mmoles), and benzene (600 mL) is kept overnight at room temperature. The solution is concentrated under vacuum, the oily residue is dissolved in methylene chloride (500 mL) and treated with 50 g of triethylamine during 4 hours at room temperature. After extraction with 2 N hydrochloric acid (6×500 mL), the organic layer is dried over sodium sulfate and discoloured by filtration through a layer of silica gel and another of carbon black. The oily residue obtained after concentration (110 g) is extracted several times with petroleum ether to remove some insoluble N-carbethoxyphthalimide. Evaporation of the petroleum ether affords a yellow oil (94 g) which is crystallized from pentane at low temperature (85 g, 77%).

NMR ($CDCl_3$): 1.77 (3H, s), 2.65 (2H, m), 3.88—5.55 (3H, complex m), 4.70 (2H, broad s), 7.72 (4H, m).

(C) 1-Fluoro-2-phthalimido-4-methylene-5-bromopentane

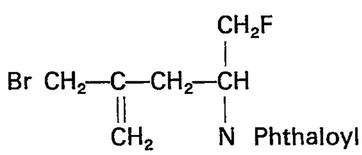

A mixture of 1-fluoro-2-phthalimido-4-methyl-4-pentene (28.3 g, 115 mmoles) prepared as in step B above, N-bromosuccinimide (20.4 g, 115 mmoles), carbontetrachloride (300 mL), and a few mgs of benzoyl peroxide is heated under strong reflux (325 W lamp) during 7.5 hours. After cooling and filtration, the solution is washed with water (100 mL, 3 times), dried over magnesium sulfate and concentrated. The oily residue (quantitative), consisting mainly of the title compound plus some 1-fluoro-2-phthalimido-4-methyl-5-bromo-3-pentene, is used for the next step without further purification.

(D) 1-Fluoro-2,5-diphthalimido-4-methylene-pentane and 1-fluoro-2,5-diphthalimido-4-methyl-3-(E,Z)-pentenes

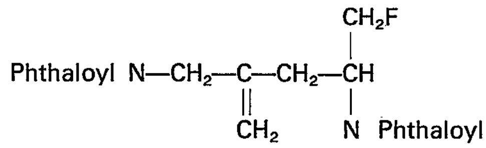

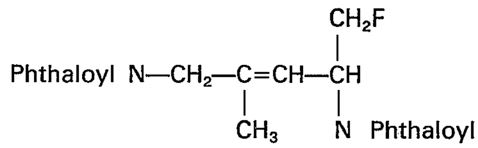

A mixture of 1-fluoro-2-phthalimido-4-methylene-5-bromo pentane (and isomers) (112 g, 345 mmoles) prepared as in step C above and potassium phthalimide (64 g, 345 mmoles) is heated at 80°C in dry N,N-dimethylformamide (DMF) (200 mL) for 5 hours. After removal of the DMF under vacuum the colored residue is dissolved in chloroform and the organic solution is successively washed with water, twice with 1 N potassium hydroxide, once with 1 N hydrochloric acid and finally twice with brine. The organic solution is dried, discolored by filtration through two layers of silica gel and charcoal, and concentrated. The yellow oil obtained (110 g) is crystallized from ether/petroleum ether to give a mixture of isomers containing mainly 1-fluoro-2,5-diphthalimido-4-methylene-pentane together with some 1-fluoro-2,5-diphthalimido-4-methyl-3-pentene (49 g). The mother liquors (59.7 g) chromatographed on silica gel (1 kg, ethyl acetate/petroleum ether 3/7) give 1-fluoro-2,5-diphthalimido-4-methyl-3-(*Z*)-pentene (4 g; 2 g after crystallization from ether), a mixture of the three title compounds (6 g) and pure 1-fluoro-2,5-diphthalimido-4-methylene-pentane (13 g).

Overall yield of the three isomers: 50%.

NMR data: 1-Fluoro-2,5-diphthalimido-4-methylene-pentane:

NMR ($CDCl_3$): 2.67 (2H, m), 3.93—5.67 (3H, complex m), 4.23 (2H, broad s), 4.93 (2H, broad s), 7.70 (8H, m).

1-Fluoro-2,5-diphthalimido-4-methyl-3-(*Z*)-pentene:

NMR ($CDCl_3$): 1.70 (3H, broad s), 4.45 (2H, AB, $J_{AB}$=8 Hz), 4.10—5.73 (3H, complex m), 5.85 (1H, m), 7.80 (8H, m).

1-Fluoro-2,5-diphthalimido-4-methyl-3-(*E*)-pentene (not obtained pure) NMR ($CDCl_3$): 1.83 (broad s, $H_3C—C—$), 5.80 (m, —C=C—H).

(E) 1-Fluoro-2,5-diamino-4-methylene-pentane, dihydrochloride and 1-fluoro-2,5-diamino-4-methyl-3-pentenes

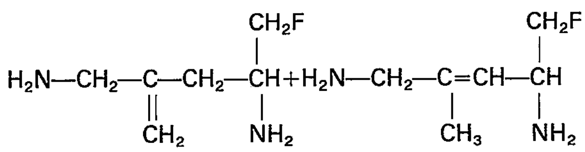

A mixture of 1-fluoro-2,5-diphthalimido-4-methylene pentane and isomers (3.93 g, 10 mmoles) obtained as in step D above and hydrazine hydrate (20 mL of a 1 molar solution in ethanol) is heated for 18 min at 90°C, and after addition of 15 mL of water and 25 mL of conc. hydrochloric acid, heated for an additional 5 min at the same temperature. After complete elimination of the excess of acid by evaporation, the residue is retreated under the same conditions as described above except that the heating with hydrazine hydrate is extended to 30 min. After dissolving the residue in water, removal of phthalhydrazide by filtration, and concentration under vacuum, the residue is dissolved in dry ethanol, and hydrazine dihydrochloride is removed by filtration. Evaporation gives a brownish oil which is used for the next step without further purification.

(F) 1-Fluoro-2,5-di-t-butoxycarbonylamino-4-methylene-pentane and 1-Fluoro-2,5-di-t-butoxycarbonyl-amino-4-methyl-3-(E)-pentene

The oil obtained as in Step E above (10 mmoles), di-t-butyl dicarbonate (5.23 g, 24 mmoles), triethylamine (3.03 g, 30 mmoles), water (6 mL), and tetrahydrofuran (30 mL) are kept at room temperature for 5 hours. After concentration and work-up with chloroform and water, 4.5 g of a colorless oil are obtained which is chromatographed on silica gel (ethyl acetate/petroleum ether: 2/8) to give 1-fluoro-2,5-di-t-butoxycarbonyl-amino-4-methylene-pentane (1.7 g, 1.34 g after crystallization from ether/petroleum ether at −4°C) followed by mixed fractions and 1-fluoro-2,5-di-t-butoxy-carbonylamino-4-methyl-3-(E)-pentene (1.08 g, 660 mg after crystallization from ether/petroleum ether). Overall yield for the 2 isomers (the

*cis*-pentene derivative is assumed to have been lost during the hydrazine hydrate treatment) is nearly quantitative.

1-Fluoro-2,5-di-tert.butoxycarbonylamino-4-methylene-pentane.

NMR ($CDCl_3$): 1.38 (18H, s), 2.25 (2H, d, J=7 Hz), 3.67 (2H, d, J=6 Hz), 4.00 (1H, broad m), 4.37 (2H, d of m, $J_{H-F}$=47 Hz), 4.90 (2H, 2-NH—, m), 4.93 (2H, m).

1-Fluoro-2,5-di-tert.butoxycarbonylamino-4-methyl-3-(E)-pentene.

NMR ($CDCl_3$): 1.43 (18H, s), 1.73 (3H, broad s), 3.65 (2H, d, J=7 Hz), 4.35 (2H, d of m, $J_{H-F}$=48 Hz), between 4.0 and 5.0 (3H, 2-NH—, broad m), 5.32 (1H, m).

(G) 1-Fluoro-2,5-diamino-4-methyl-3-(E)-pentene, dihydrochloride

1-Fluoro-2,5-di-t-butoxycarbonylamino-4-methyl-3-(E)-pentene (650 mg, 1.96 mmole) obtained as in step F above is dissolved in dry ether saturated with hydrogen chloride gas. After standing overnight at room temperature, the white solid obtained by decantation is recrystallized from methanol/ether (320 mg, 80%).

NMR ($D_2O/DCl$): 1.85 (3H, broad s), 3.62 (2H, narrow m), 4.53 (1H, broad m), 4.62 (2H, d of m, $J_{H-F}$=46 Hz), 5.52 (1H, m).

Anal. Calcd for $C_6H_{13}N_2F.2HCl$: C, 35.14; H, 7.37; N, 13.66 Found: C, 35.25; H, 7.13; N, 13.66.

(H) 1-Fluoro-2,5-diamino-4-methylene-pentane, dihydrochloride

1-Fluoro-2,5-di-t-butoxycarbonylamino-4-methylene-pentane (650 mg, 1.95 mmole) obtained as in step F above is dissolved in dry ether saturated with HCl gas. After standing overnight at room temperature, the white solid obtained is recrystallized from methanol/ether (350 mg, 87%).

NMR ($D_2O/DCl$): 2.75 (2H, d, J=8 Hz), 3.68 (2H, broad s), 3.97 (1H, broad m), 4.72 (2H, d of m, $J_{HF}$=48 Hz), 5.42 (2H, broad s).

Anal. Calcd for $C_6H_{13}N_2F.2HCl$: C, 35.14; H, 7.37; N, 13.66 Found: C, 35.15; H, 7.14; N, 13.69.

Example II

1-Fluoro-2,5-diamino-3-methyl-3-(E)-pentene, dihydrochloride

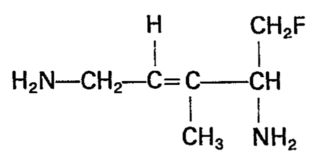

A) Preparation of:—

2-Bromo-4-ethoxy-2-butene

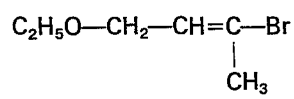

A freshly prepared solution of sodium ethoxide in dry ethanol (6.9 g Na, 0.3 mole, 100 ml EtOH) is added under nitrogen to 2,4-dibromo-2-butene (59 g, 0.275 mole) in 20 ml of dry ethanol. After 1.5 hours at room temperature, 100 ml of water is added to the reaction mixture, the product is extracted twice with small portions of petroleum ether and dried over magnesium sulfate. Distillation affords 2-bromo-4-ethoxy-2-butene.

B) Preparation of:—

1-Fluoro-2-amino-3-methyl-5-ethoxy-3-pentene

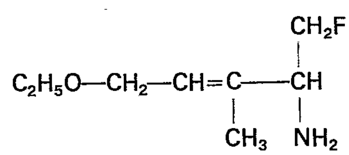

Under an atmosphere of nitrogen, 4-ethoxy-2-butene-2-yl-magnesium bromide is prepared from 8.25 g of 2-bromo-4-ethoxy-2-butene (50 mmoles) prepared as in Step A above, 12.15 g of magnesium turnings (500 mmoles) and 50 ml of dry tetrahydrofuran. After 4 hours the Grignard solution is transferred into another flask via a syringe, cooled to −30°C and fluoroacetonitrile (2.36 g, 40 mmoles) in tetrahydrofuran (30 ml) is added dropwise during 15 mins. After 15 additional minutes at −30°C, a solution/suspension of sodium borohydride (1.52 g, 40 mmoles) in methanol (100 ml) and water (2 ml) cooled to −50°C is poured into the reaction mixture previously cooled to −50°C. The temperature rises up to −30°C, and after stirring

for 20 mins at −20°C, the mixture is allowed to warm up to 0°C during 1 hour. After acidifying with 6 N hydrochloric acid and evaporation, the residue is extracted twice with diethyl ether to remove by-products, made alkaline with 4 N sodium hydroxide and extracted twice with diethyl ether. Evaporation of the solvent affords crude 1-fluoro-2-amino-3-methyl-5-ethoxy-3-pentene.

C) Preparation of:
N-1-Fluoro-3-methyl-5-ethoxy-3-pentene-2-yl, $N^1$-ethoxycarbonyl-o-phthalamide

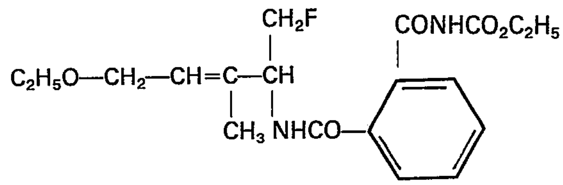

A mixture of 1-fluoro-2-amino-3-methyl-5-ethoxy-3-pentene (1 g, 6.8 mmoles) prepared as in Step B above, N-ethoxycarbonylphthalimide (1.49 g, 6.8 mmoles) and 25 ml of dry benzene is kept over night at room temperature. The N-1-fluoro-3-methyl-5-ethoxy-3-pentene-2-yl, $N^1$-ethoxycarboxyl-*o*-phthalimide is isolated by evaporation of the solvent and is used for the following Step D, without purification.

D) Preparation of:—
1-Fluoro-2-phthalimido-3-methyl-5-ethoxy-3-pentene

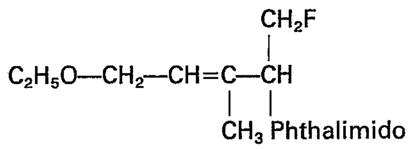

Treatment of N-1-fluoro-3-methyl-5-ethoxy-3-pentene-2-yl, N′-ethoxycarbonyl-*o*-phthalimide prepared as in Step C above with triethylamine (687 mg, 6.8 mmoles) in methylene chloride for 5 hours at room temperature followed by 2 extractions with 1 N hydrochloride acid and evaporation gives crude 1-fluoro-2-phthalimido-3-methyl-5-ethoxy-3-pentene.

Rapid chromatography on silica (ethyl acetate:petroleum ether 15:85) gives three fractions: fraction A (150 mg), a mixed fraction B (385 mg) and fraction C (320 mg), A and C representing respectively pure cis-1-fluoro-2-phthalimido-3-methyl-5-ethoxy-3-pentene and trans-1-fluoro-2-phthalimido-3-methyl-5-ethoxy-3-pentene.

E) Preparation of:—
1-Fluoro-2-phthalimido-3-methyl-5-bromo-3-(E)-pentene

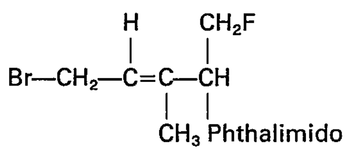

Boron tribromide (106 mg, 0.42 mmoles) in 5 ml of dry methylene chloride is added slowly to a solution of 1-fluoro-2-phthalimido-3-methyl-5-ethoxy-3-(E)-pentene (i.e. trans), 336 mg, 1.15 mmoles) prepared as in Step D above in 10 ml of dry methylene chloride cooled at −78°C. The temperature is allowed to rise to room temperature overnight, the solvent is evaporated, and 1-fluoro-2-phthalimido-3-methyl-5-bromo-3-(E)-pentene is obtained.

F) Preparation of:—
1-Fluoro-2,5-diphthalimido-3-methyl-3-(E)-pentene

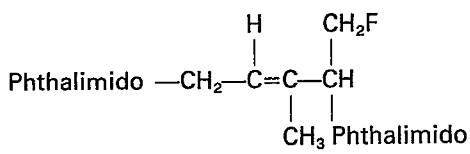

A mixture of 1-fluoro-2-phthalimido-3-methyl-5-bromo-3-(E)-pentene (360 mg, 1.10 mmole) prepared as in Step E above and potassium phthalimide (245 mg, 1.32 mmole) is heated at 80°C in dry

N,N-dimethylformamide (5 ml) for 2.5 hours. After cooling, water is added to the reaction mixture and the solid is filtered off. Chloroform/1 N potassium hydroxide extraction to remove the excess of phthalimide, drying, filtration and evaporation of the solvent afford 1-fluoro-2,5-diphthalimido-3-methyl-3-(E)-pentene.

G) Preparation of:—
1-Fluoro-2,5-diamino-3-methyl-3-(E)-pentene

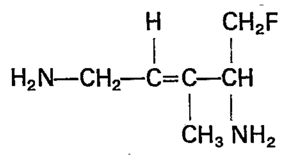

1-Fluoro-2,5-diphthalimido-3-methyl-3-(E)-pentene (10.5 g; 27.7 mmoles) prepared as in Step F above is heated at 95°C in concentrated hydrochloric acid (250 ml) and acetic acid (100 ml during 24 hours. After evaporation of the solvent, the residue is taken up in water and phthalic acid is filtered off. The filtrate is evaporated and the solid residue is crystallized from methanol-acetone to give 1-fluoro-2,5-diamino-3-methyl-3-(E)-pentene, dihydrochloride (4.2 g; 79%).

The dihydrochloride salt is dissolved in methanol, sodium methoxide (2 equivalents) added and the solution evaporated to dryness under reduced pressure. The residue is dissolved in absolute ethanol, filtered and the filtrate evaporated to dryness under reduced pressure to yield 1-fluoro-2,5-diamino-3-methyl-3-(E)-pentene.

The following Examples relating to pharmaceutical compositions, the term "active compound" is used to indicate the compound 1-fluoro-2,5-diamino-4-methyl-3-(E)-pentene. This compound may be replaced in these compositions by any other compound of the invention, for example by 1-fluoromethyl-2,5-diamino-3-methyl-3-(E)-pentene. Adjustments in the amount of medicament may be necessary or desirable depending upon the degree of activity of the medicament as is well known in the art.

Example III

An illustrative composition for hard gelatin capsules is as follows:—

| | |
|---|---|
| (a) active compound | 20 mg |
| (b) talc | 5 mg |
| (c) lactose | 90 mg |

The formulation is prepared by passing the dry powders of (a) and (b) through a fine mesh screen and mixing them well. The powder is then filled into hard gelatine capsules at a net fill of 115 mg per capsule.

Example IV

An illustrative composition for tablets is as follows:—

| | |
|---|---|
| (a) active compound | 20 mg |
| (b) starch | 43 mg |
| (c) lactose | 45 mg |
| (d) magnesium stearate | 2 mg |

The granulation obtained upon mixing the lactose with the compound (a) and part of the starch and granulated with starch paste is dried, screened, and mixed with the magnesium stearate. The mixture is compressed into tablets weighing 110 mg each.

Example V

An illustrative composition for an injectable suspension is the following 1 ml ampul for an intramuscular injection:—

| | weight per cent |
|---|---|
| (a) active compound | 1.0 |
| (b) polyvinylpyrrolidone | 0.5 |
| (c) lecithin | 0.25 |
| (d) water for injection to make | 100.0 |

The materials (a)—(d) are mixed, homogenized, and filled into 1 ml ampuls which are sealed and autoclaved 20 minutes at 121°C. Each ampul contains 10 mg per ml of novel compound (a).

Example VI

| | mg/suppository |
|---|---|
| Active Compound | 50 |
| Oil of Theobroma | 950 |

The medicament is powdered and passed through a B.S. No. 100 sieve and triturated with molten oil of Theobroma at 45°C to form a smooth suspension. The mixture is well stirred and poured into moulds each of nominal 1G capacity, to produce suppositories.

Example VII

The ODC inhibitory activity of the compounds of Formula I can be demonstrated *in vivo* according to the following procedure:

Male rats of the Sprague-Dawley strain (200—220 g body weight), purchased from Charles River, are given food and water *ad libitum* under a constant 12 hr light—12 hr dark lighting schedule. Drugs are injected intraperitoneally (dissolved in 0.9% saline) or are given by gavage (dissolved in water). Rats given saline or water serve as control. Five to six hours after drug administration, the animals are killed by decapitation and the ventral prostate, testis and thymus are excised rapidly and immediately processed. The tissues are homogenized with three volumes of 30 mM sodium phosphate buffer (pH 7.1) containing 0.1 mM EDTA, 0.25 M sucrose, 0.1 mM pyridoxal phosphate and 5 mM dithiothreitol. Ornithine decarboxylase activities are determined on a 1000 g supernatant of testis or prostate homogenate and on a whole thymus homogenate, essentially as described by Ono *et al* (Biochem. Biophys. Acta, *284*, 285 (1972)).

When tested according to the above-described procedure, the compound of Example I gave the results shown below at a single oral dose of 25 mg/kg body weight and 6 hours after administration.

| ODC activity (% control) | | |
|---|---|---|
| Ventral prostate | Thymus | Testis |
| 36 | 46 | 53 |

Example VIII

The activity of the compounds of Formula I as inhibitors of ornithine decarboxylase (ODC) can be demonstrated *in vitro* according to the following procedure:

Ornithine decarboxylase (ODC) is prepared from the livers of rats which have been injected with thioacetamide (150 mg/kg of body weight) 18 hrs before sacrifice, and is purified about ten fold by acid treatment at pH 4.6 as described by Ono *et al* (Biochem. Biophys, Acta *284*, 285 (1972)). The stock solution of ODC is comprised of protein (16 mg/mL), sodium phosphate buffer (30 mM, pH 7.1), dithiothreitol (5 mM) and pyridoxal phosphate (0.1 mM). The specific activity of this stock solution is 0.12 nmol of $CO_2$/min per mg of protein. For a typical experiment 320 µl of this stock solution are mixed at time 0 with 80 µl of a solution of the inhibitor in water and incubated at 37°. At different times 50 µl aliquots are transferred into a 1-mL assay medium containing sodium phosphate (30 mM, pH 7.1), dithiothreitol (5 mM), pyridoxal phosphate (0.1 mM), L-ornithine (0.081 µmol), and DL-[1-$^{14}$C] ornithine (0.043 µmol, 58 Ci/mol, Amersham) in a closed vessel in which a filter paper moistered with 50 µl hyamine hydroxide (1 M) is fitted. The reaction is allowed to proceed for 60 min at 37°C and then terminated by addition of 0.5 ml of 40% trichloroacetic acid. After an additional 30 min the $CO_2$ absorbed on the filter paper is counted in a standard scintillation cocktail. $K_I$ (apparent dissociation constant) and $\gamma_{50}$ (half-life, at infinite concentration of inhibitor are calculated according to the method of Kitz and Wilson (J. Biol. Chem., *237*, 3245 (1962)).

When tested according to the above-described procedure, the compound of Example I gave the results shown in below.

Half-life ($t_{1/2}$) at 10 μM is also set forth.

TABLE III

| ODC | | |
|---|---|---|
| $K_I$ (μM) | $\gamma_{50}$ (Min.) | $t_{1/2}$ (Min.) |
| 8.5 | 4.5 | 7.8 |

**Claims for the Contracting States: BE CH DE FR IT LI LU NL SE**

1. A fluorinated alkenylene diamine derivative of the following general Formula I:—

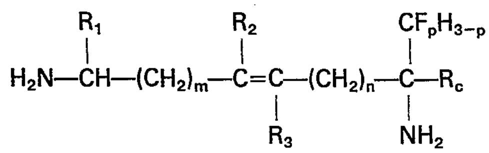

Formula I

wherein:—

$R_c$ represents hydrogen or $-COR_5$, where $R_5$ is as defined below;
$R_1$ represents hydrogen or $C_1-C_6$ alkyl;
one of $R_2$ and $R_3$ represents hydrogen and the other represents $C_1-C_6$ alkyl;
$R_5$ represents hydroxy or $C_1-C_8$ alkoxy;
*m* and *n* independently represent 0 or 1 but $m+n=0$ or 1;
*p* represents 1 or 2 or a pharmaceutically acceptable salt thereof.

2. A compound as claimed in Claim 1 and having the following general Formula IA:—

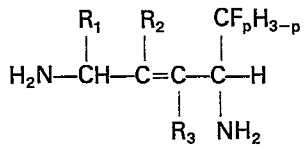

Formula IA

wherein $R_1$, $R_2$, $R_3$, and *p* are as defined in Claim 1; or a pharmaceutically acceptable salt thereof.

3. A compound as claimed in Claim 1 and having the following general Formula IB:—

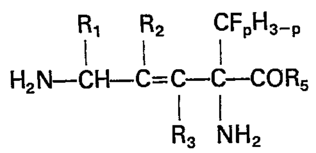

Formula IB

wherein $R_1$, $R_2$, $R_3$, $R_5$, and *p* are as defined in Claim 1; or a pharmaceutically acceptable salt thereof.

4. A compound as claimed in any one of Claims 1 to 3 wherein $R_2$ represents $C_1-C_6$ alkyl and $R_3$ represents hydrogen.

5. A compound as claimed in Claim 4 wherein $R_2$ represents methyl.

6. A compound as claimed in any one of Claims 1 to 3 wherein $R_3$ represents $C_1-C_6$ alkyl and $R_2$ represents hydrogen.

7. A compound as claimed in Claim 6 wherein $R_3$ represents methyl.

8. A compound as claimed in any one of Claims 1 to 7 wherein $R_1$ represents hydrogen.

9. A compound as claimed in any one of Claims 1 and 3 to 7 wherein $R_c$ represents carboxy.

10. A compound as claimed in any one of Claims 1 and 3 to 7 wherein $R_c$ represents $C_2-C_9$ alkoxycarbonyl.

11. A compound as claimed in any one of Claims 1 to 10 wherein *p* is 1.

12. 1-Fluoro-2,5-diamino-3-methyl-3-(E)-pentene or a pharmaceutically acceptable salt thereof.

13. 1-Fluoro-2,5-diamino-4-methyl-3-(E)-pentene or a pharmaceutically acceptable salt thereof.

14. A compound as claimed in any one of the preceding claims for use in a method of treatment of the human or animal body by therapy or of diagnosis practiced on the human or animal body.

15. A compound as claimed in any one of the preceding claims for use in the inhibition in the human or animal body of ornithine decarboxylase.

16. A pharmaceutical composition comprising a compound as claimed in any one of the preceding

claims in admixture or otherwise in association with a pharmaceutically acceptable carrier or diluent therefor.

17. A pharmaceutical composition as claimed in Claim 16 in unit dosage form containing 10 mg to 300 mg of said compound per unit dose.

18. A method of preparing a compound as claimed in Claim 1 which comprises amination in manner known *per se* of an amino-protected derivative of a compound of the following general Formula II:—

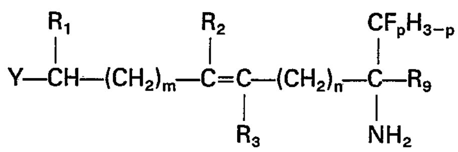

Formula II

wherein:

$R_1$, $R_2$, $R_3$, *m*, *n* and *p* are as defined in Claim 1;

$R_9$ represents hydrogen, cyano or $C_2$—$C_9$ alkoxycarbonyl; and

Y represents a leaving group and, when $R_9$ is cyano, converting the cyano group into a carboxy group and, if necessary, subsequently removing the amino-protecting group(s).

19. A method of preparing a compound as claimed in Claim 1 in which *m* is 0, $R_2$ is methyl and $R_c$ is carboxy which comprises acid hydrolysis of a di-phthalimido derivative of a compound of the following general Formula XII:—

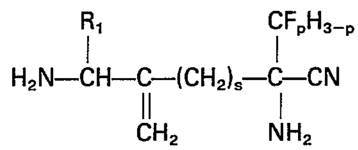

Formula XII

wherein:—

$R_1$, and *p* are as defined in Claim1; and *s* is 1 or 2.

**Claims for the Contracting State: AT**

1. A method of preparing a pharmaceutical composition by admixing or otherwise associating a pharmacologically active compound with a pharmaceutically acceptable carrier or diluent, characterised in that said compound is a fluorinated alkenylene diamine derivative of the following general Formula I:—

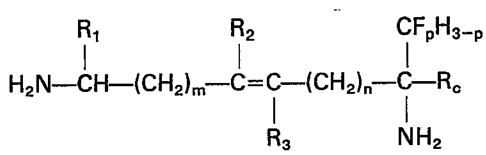

Formula I

wherein:—

$R_c$ represents hydrogen or —$COR_5$, where $R_5$ is as defined below;

$R_1$ represents hydrogen or $C_1$—$C_6$ alkyl;

one of $R_2$ and $R_3$ represents hydrogen and the other represents $C_1$—$C_6$ alkyl;

$R_5$ represents hydroxy or $C_1$—$C_8$ alkoxy;

*m* and *n* independently represent 0 or 1 but $m+n=0$ or 1;

*p* represents 1 or 2 or a pharmaceutically acceptable salt thereof.

2. A method as claimed in Claim 1, wherein said compound has the following general Formula IA:—

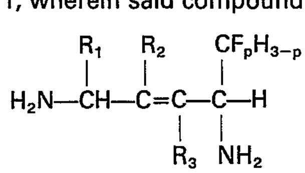

Formula IA

wherein $R_1$, $R_2$, $R_3$, and *p* are as defined in Claim 1.

3. A method as claimed in Claim 1, wherein said compound has the following general Formula IB:—

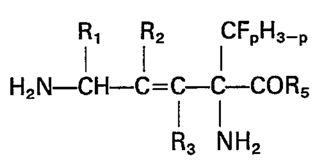

Formula IB

wherein $R_1$, $R_2$, $R_3$, $R_5$, and *p* are as defined in Claim 1.

4. A method as claimed in any one of Claims 1 to 3, wherein $R_2$ represents $C_1$—$C_6$ alkyl and $R_3$ represents hydrogen.

5. A method as claimed in Claim 4, wherein $R_2$ represents methyl.

6. A method as claimed in any one of Claims 1 to 3, wherein $R_3$ represents $C_1$—$C_6$ alkyl and $R_2$ represents hydrogen.

7. A method as claimed in Claim 6, wherein $R_3$ represents methyl.

8. A method as claimed in any one of Claims 1 to 7, wherein $R_1$ represents hydrogen.

9. A method as claimed in any one of Claims 1 and 3 to 7, wherein $R_c$ represents carboxy.

10. A method as claimed in any one of Claims 1 and 3 to 7, wherein $R_c$ represents $C_2$—$C_9$ alkoxycarbonyl.

11. A method as claimed in any one of Claims 1 to 10, wherein *p* is 1.

12. A method of preparing a compound as defined in Claim 1 which comprises amination in manner known *per se* of an amino-protected derivative of a compound of the following general Formula II:—

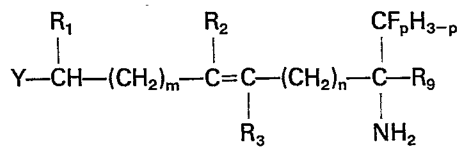

Formula II

wherein:

$R_1$, $R_2$, $R_3$, *m*, *n* and *p* are as defined in Claim 1;

$R_9$ represents hydrogen, cyano or $C_2$—$C_9$ alkoxycarbonyl; and

Y represents a leaving group and, when $R_9$ is cyano, converting the cyano group into a carboxy group and, if necessary, subsequently removing the amino-protecting group(s).

13. A method of preparing a compound as defined in Claim 1 in which *m* is 0, $R_2$ is methyl and $R_c$ is carboxy which comprises acid hydrolysis of a di-phthalimido derivative of a compound of the following general Formula XII:—

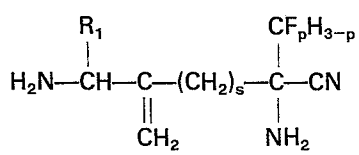

Formula XII

wherein:—

$R_1$, and *p* are as defined in Claim 1; and *s* is 1 or 2.

**Patentansprüche für die Vertragsstaaten: BE, CH, DE, FR, IT, LI, LU, NL, SE**

1. Fluoriertes Alkylendiamin-Derivat der folgenden allgemeinen Formel I

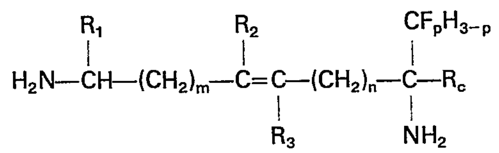

(I)

in der

$R_c$ Wasserstoff oder —$COR_5$ bedeutet, wobei $R_5$ wie nachstehende definiert ist;

$R_1$ Wasserstoff oder einen $C_1$—$C_6$-Alkylreste bedeutet;

einer der Reste $R_2$ und $R_3$ Wasserstoff und der andere einen $C_1$—$C_6$-Alkylrest darstellt;

$R_5$ eine Hydroxylgruppe oder ein $C_1$—$C_8$-Alkoxyrest bedeutet;

m und n unabhängig voneinander den Wert 0 oder 1 haben, jedoch $m+n=0$ oder 1 ist;

p den Wert 1 oder 2 hat, oder ein pharmazeutisch verträgliches Salz davon.

2. Verbindung nach Anspruch 1 der folgenden allgemeinen Formel IA

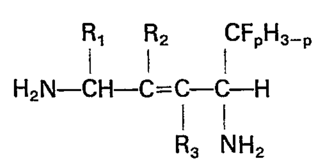

(IA)

in der $R_1$, $R_2$, $R_3$ und p wie in Anspruch 1 definiert sind, oder ein pharmazeutisch verträgliches Salz davon.

3. Eine Verbindung nach Anspruch 1 oder folgenden allgemeinen Formel IB

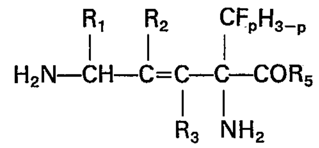

(IB)

in der $R_1$, $R_2$, $R_3$, $R_5$ und p wie in Anspruch 1 definiert sind, oder ein pharmazeutisch verträgliches Salz davon.

4. Verbindung nach einem der Ansprüche 1 bis 3, wobei $R_2$ einen $C_1$—$C_6$-Alkylrest bedeutet und $R_3$ Wasserstoff darstellt.

5. Verbindung nach Anspruch 4, wobei $R_2$ eine Methylgruppe bedeutet.

6. Verbindung nach einem der Ansprüche 1 bis 3, wobei $R_3$ einen $C_1$—$C_6$-Alkylrest bedeutet und $R_2$ Wasserstoff darstellt.

7. Verbindung nach Anspruch 6, wobei $R_3$ die Methylgruppe bedeutet.

8. Verbindung nach einem der Ansprüche 1 bis 7, wobei $R_1$ Wasserstoff bedeutet.

9. Verbindung nach einem der Ansprüche 1 und 3 bis 7, wobei $R_c$ eine Carboxylgruppe bedeutet.

10. Verbindung nach einem der Ansprüche 1 und 3 bis 7, wobei $R_c$ einen $C_2$—$C_9$-Alkoxycarbonylrest darstellt.

11. Verbindung nach einem der Ansprüche 1 bis 10, wobei p den Wert 1 hat.

12. 1-Fluor-2,5-diamino-3-methyl-3-(E)-penten oder ein pharmazeutisch verträgliches Salz davon.

13. 1-Fluor-2,5-diamino-4-methyl-3-(E)-penten oder ein pharmazeutisch verträgliches Salz davon.

14. Verbindung nach einem der vorangehenden Ansprüche zur Verwendung in einem Verfahren zur therapeutischen oder diagnostischen, am menschlichen oder tierischen Körper durchgeführten Verfahren zur Behandlung des menschlichen oder tierischen Körpers.

15. Verbindung nach einem der vorangehenden Ansprüche zur Verwendung bei der Inhibierung der Ornithin-Decarboxylase im menschlichen oder tierischen Körper.

16. Arzneimittel, umfassend eine Verbindung nach einem der vorangehenden Ansprüche im Gemisch oder eine anderweitige Verbindung mit einem pharmazeutisch verträglichen Träger oder Verdünnungsmittel dafür.

17. Arzneimittel nach Anspruch 16 in Dosierungseinheitsform, enthaltend 10 bis 300 mg der genannten Verbindung pro Einheitsdosis.

18. Verfahren zur Herstellung einer Verbindung nach Anspruch 1, dadurch gekennzeichnet, daß man in an sich bekannter Weise ein an der Aminogruppe geschütztes Derivat eine Verbindung der folgenden allgemeinen Formel II

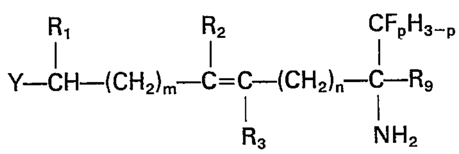

(II)

in der

$R_1$, $R_2$, $R_3$, m, n und p wie in Anspruch 1 definiert sind,

$R_9$ Wasserstoff, eine Cyanogruppe oder einen $C_2$—$C_9$-Alkoxycarbonylrest bedeutet, und

Y eine austretende Gruppe darstellt, in an sich bekannter Weise aminiert und, wenn $R_9$ die Cyanogruppe ist, die Cyanogruppe in eine Carboxylgruppe umwandelt, und, falls erforderlich, anschließend die Amino-Schutzgruppe(n) abspaltet.

19. Verfahren zur Herstellung einer verbindung nach Anspruch 1, in der m den Wert 0 hat, $R_2$ eine Methylgruppe und $R_c$ eine Carboxylgruppe ist, gekennzeichnet durch saure Hydrolyse eines Diphthalimido-Derivates einer Verbindung der folgenden allgemeinen Formel XII

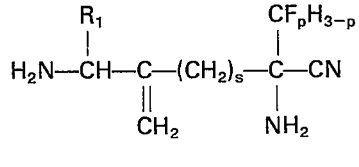

(XII)

in der $R_1$ und p wie in Anspruch 1 definiert sind und s den Wert 1 oder 2 hat.

**Patentansprüche für den Vertragsstaat: AT**

1. Verfahren zur Herstellung eines Arzneimittels, wobei eine pharmakologisch aktive Verbindung mit einem pharmazeutisch verträglichen Träger oder Verdünnungsmittel vermischt oder in anderer Weise

verbunden wird, dadurch gekennzeichnet, daß die genannte Verbindung ein fluoriertes Alkylendiamin-Derivat der folgenden allgemeinen Formel I

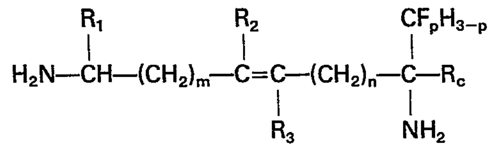

(I)

in der

$R_c$ Wasserstoff oder $—COR_5$ bedeutet, wobei $R_5$ wie nachstehend definiert ist;

$R_1$ Wasserstoff oder einen $C_1—C_6$-Alkylrest bedeutet; einer der Reste $R_2$ und $R_3$ Wasserstoff und der andere einen $C_1—C_6$-Alkylrest darstellt;

$R_5$ eine Hydroxylgruppe oder ein $C_1—C_8$-Alkoxyrest bedeutet;

m und n unabhängig voneinander den Wert 0 oder 1 haben, jedoch $m+n=0$ oder 1 ist;

p den Wert 1 oder 2 hat oder ein pharmazeutisch verträgliches Salz davon ist.

2. Verfahren nach Anspruch 1, wobei die genannte Verbindung die folgende allgemeine Formel IA aufweist

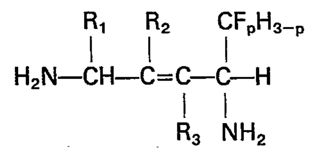

(IA)

in der $R_1$, $R_2$, $R_3$ und p wie in Anspruch 1 definiert sind.

3. Verfahren nach Anspruch 1, wobei die genannte Verbindung die folgende allgemeine Formel IB aufweist

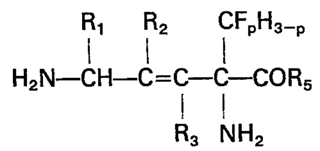

(IB)

in der $R_1$, $R_2$, $R_3$, $R_5$ und p wie in Anspruch 1 definiert sind.

4. Verfahren nach einem der Ansprüche 1 bis 3, wobei $R_2$ einen $C_1—C_6$-Alkylrest bedeutet und $R_3$ Wasserstoff darstellt.

5. Verfahren nach Anspruch 4, wobei $R_2$ eine Methylgruppe bedeutet.

6. Verfahren nach einem der Ansprüche 1 bis 3, wobei $R_3$ einen $C_1—C_6$-Alkylrest bedeutet und $R_2$ Wasserstoff darstellt.

7. Verfahren nach Anspruch 6, wobei $R_3$ eine Methylgruppe bedeutet.

8. Verfahren nach einem der Ansprüche 1 bis 7, wobei $R_1$ Wasserstoff bedeutet.

9. Verfahren nach einem der Ansprüche 1 und 3 bis 7, wobei $R_c$ eine Carboxylgruppe bedeutet.

10. Verfahren nach einem der Ansprüche 1 und 3 bis 7, wobei $R_c$ einen $C_2—C_9$-Alkoxycarbonylrest bedeutet.

11. Verfahren nach einem der Ansprüche 1 bis 10, wobei p den Wert 1 hat.

12. Verfahren zur Herstellung einer Verbindung nach Anspruch 1, dadurch gekennzeichnet, daß man in an sich bekannter Weise ein an der Aminogruppe geschütztes Derivat einer Verbindung der folgenden allgemeinen Formel II

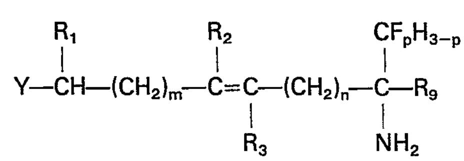

(II)

in der

$R_1$, $R_2$, $R_3$, m, n und p wie in Anspruch 1 definiert sind,

$R_9$ Wasserstoff, eine Cyanogruppe oder einen $C_2—C_9$-Alkoxycarbonylrest bedeutet, und

Y eine austretende Gruppe darstellt, in an sich bekannter Weise aminiert und, wenn $R_9$ die Cyanogruppe ist, die Cyanogruppe in eine Carboxylgruppe umwandelt, und, falls erforderlich, anschließend die Amino-Schutzgruppe(n) abspaltet.

13. Verfahren zur Herstellung einer Verbindung nach Anspruch 1, in der m den Wert 0 hat, $R_2$ eine

Methylgruppe und $R_c$ eine Carboxylgruppe ist, gekennzeichnet durch saure Hydrolyse eines Diphthalimido-Derivates einer Verbindung der folgenden allgemeinen Formel XII

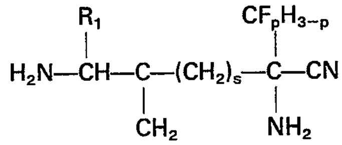

(XII

in der $R_1$ und p wie in Anspruch 1 definiert sind und s den Wert 1 oder 2 hat.

**Revendications pour les Etats Contractants: BE, CH, DE, FR, IT, LI, LU, NL, SE**

1. Un dérivé fluoré d'alcénylènediamine répondant à la formule générale I suivante:

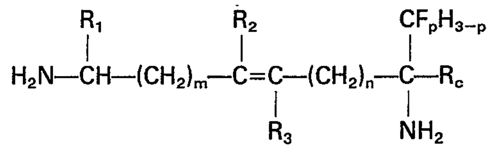

Formule I

dans laquelle:

$R_c$ représente un hydrogène ou $—COR_5$, où $R_5$ est comme défini ci-dessous;
$R_1$ représente un hydrogène ou un alkyle en $C_1—C_6$;
un de $R_2$ et $R_3$ représente un hydrogène et l'autre représente un alkyle en $C_1—C_6$;
$R_5$ représente un hydroxy ou un alkoxy en $C_1—C_8$;
m et n représentent indépendamment 0 ou 1 mais m+n=0 ou 1;
p représente 1 ou 2; ou un sel acceptable en pharmacie correspondant.

2. Un composé comme revendiqué dans la revendication 1, et répondant à la formule générale IA suivante:

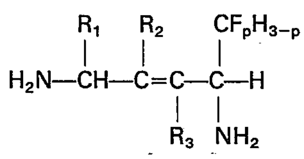

Formule IA

dans laquelle $R_1$, $R_2$, $R_3$ et p sont somme défini dans la revendication 1; ou un sel acceptable en pharmacie correspondant.

3. Un composé comme revendiqué dans la revendication 1, répondant à la formule générale IB suivante:

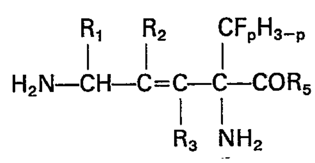

Formule IB

dans laquelle $R_1$, $R_2$, $R_3$, $R_5$ et p sont comme défini dans la revendication 1; ou un sel acceptable en pharmacie correspondant.

4. Un composé comme revendiqué dans l'une quelconque des revendications 1 à 3 où $R_2$ représente un alkyle en $C_1—C_6$ et $R_3$ représente un hydrogène.

5. Un composé comme revendiqué dans la revendication 4 où $R_2$ représente un méthyle.

6. Un composé comme revendiqué dans l'une quelconque des revendications 1 à 3 où $R_3$ représente un alkyle en $C_1—C_6$ et $R_2$ représente un hydrogène.

7. Un composé comme revendiqué dans la revendication 6 où $R_3$ représente un méthyle.

8. Un composé comme revendiqué dans l'une quelconque des revendications 1 à 7 où $R_1$ représente un hydrogène.

9. Un composé comme revendiqué dans l'une quelconque des revendications 1 et 3 à 7, où $R_c$ représente un carboxy.

10. Un composé comme revendiqué dans l'une quelconque des revendications 1 et 3 à 7, où $R_c$ représente un alcoxy-carbonyle en $C_2—C_9$.

11. Un composé comme revendiqué dans l'une quelconque des revendications 1 à 10 où p est 1.

12. 1-fluoro-2,5-diamino-3-méthyl-3-(E)-pentène ou un de ses sels acceptables en pharmacie.

13. 1-fluoro-2,5-diamino-4-méthyl-3-(E)-pentène ou un de ses sels acceptables en pharmacie.

14. Un composé comme revendiqué dans l'une quelconque des revendications précédentes, pour l'emploi dans un procédé de traitement de l'organisme de l'homme ou d'un animal par thérapeutique ou de diagnostic effectué sur l'organisme de l'homme ou d'un animal.

15. Un composé comme revendiqué dans l'une quelconque des revendications précédentes, pour l'emploi dans l'inhibition de l'ornithine-décarboxylase dans l'organisme de l'homme ou d'un animal.

16. Une composition pharmaceutique comprenant un composé comme revendiqué dans l'une quelconque des revendications précédentes, mélangé ou associé d'autre façon à un support ou diluant acceptable en pharmacie.

17. Une composition pharmaceutique comme revendiqué dans la revendication 16 sous forme d'une dose unitaire contenant 10 mg à 300 mg dudit composé par dose unitaire.

18. Un procédé de préparation d'un composé comme revendiqué dans la revendication 1 qui comprend l'amination de façon connue en soi d'un dérivé amino protégé d'un composé répondant à la formule générale II suivante:

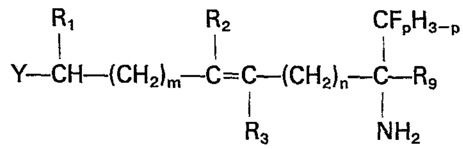

Formule II

dans laquelle:

$R_1$, $R_2$, $R_3$, m, n et p sont comme défini dans la revendication 1;

$R_9$ représente un hydrogène, un cyano ou un alcoxycarbonyle en $C_2$—$C_9$; et

Y représente un groupe labile, et, lorsque $R_9$ est un cyano, la conversion du radical cyano en un radical carboxy, et, au besoin, l'élimination ultérieure du ou des groupes amino-protecteurs.

19. Un procédé de préparation d'un composé comme revendiqué dans la revendication 1, où m est zéro, $R_2$ est un méthyle et $R_c$ est un carboxy qui comprend l'hydrolyse acide d'un dérivé diphtalimido d'un composé répondant à la formule générale XII suivante:

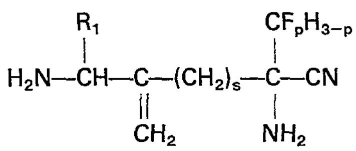

Formule XII

dans laquelle:

$R_1$ et p sont comme défini dans la revendication 1; et s est 1 ou 2.

**Revendications pour l'Etat Contractant: AT**

1. Un procédé de préparation d'une composition pharmaceutique par mélange ou association d'autre façon d'un composé à activité pharmacologique avec un support ou diluant acceptables en pharmacie, caractérisé en ce que ledit composé est un dérivé fluoré d'alcénylènediamine répondant à la formule générale I suivante:

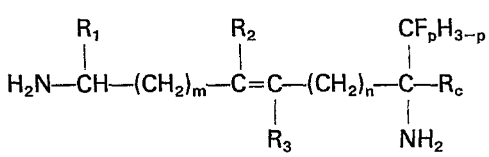

Formule I

dans laquelle:

$R_c$ représente un hydrogène ou —$COR_5$ où $R_5$ est comme défini ci-dessous;

$R_1$ représente un hydrogène ou un alkyle en $C_1$—$C_6$;

un de $R_2$ et $R_3$ représente un hydrogène et l'autre représente un alkyle en $C_1$—$C_6$;

$R_5$ représente un hydroxy ou un alcoxy en $C_1$—$C_8$;

m et n indépendamment représentent 0 ou 1 mais m+n=0 ou 1;

p représente 1 ou 2; ou un sel acceptable en pharmacie correspondant.

2. Un procédé comme revendiqué dans la revendication 1 dans lequel ledit composé répond à la formule générale IA suivante:

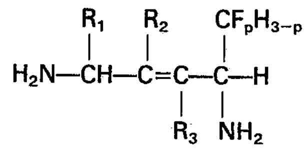

Formule IA

dans laquelle $R_1$, $R_2$, $R_3$ et p sont comme défini dans la revendication 1.

3. Un procédé comme revendiqué dans la revendication 1, dans lequel ledit composé répond à la formule générale IB suivante:

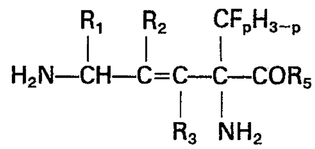

Formule IB

dans laquelle $R_1$, $R_2$, $R_3$, $R_5$ et p sont comme défini dans la revendication 1.

4. Un procédé comme revendiqué dans l'une quelconque des revendications 1 à 3 où $R_2$ représente un alkyle en $C_1$—$C_6$ et $R_3$ représente un hydrogène.

5. Un procédé comme revendiqué dans la revendication 4 où $R_2$ représente un méthyle.

6. Un procédé comme revendique dans l'une quelconque des revendications 1 à 3 où $R_3$ représente un alkyl en $C_1$—$C_6$ et $R_2$ représente un hydrogène.

7. Un procédé comme revendiqué dans la revendication 6 ou $R_3$ représente un méthyle.

8. Un procédé comme revendiqué dans l'une quelconque des revendications 1 à 7 où $R_1$ représente un hydrogène.

9. Un procédé comme revendiqué dans l'une quelconque des revendications 1 et 3 à 7 où $R_7$ représente un carboxy.

10. Un procédé comme revendiqué dans l'une quelconque des revendications 1 et 3 à 7 où $R_c$ représente un alcoxy-carbonyle en $C_2$—$C_9$.

11. Un procédé comme revendiqué dans l'une quelconque des revendications 1 à 10, où p est 1.

12. Un procédé de préparation d'un composé comme défini dans la revendication 1 qui comprend l'amination de façon connue en soi d'un dérivé amino protégé d'un composé répondant à la formule générale II suivante:

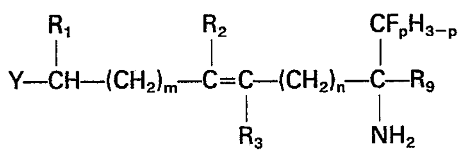

Formule II

dans laquelle:

$R_1$, $R_2$, $R_3$, m, n et p sont comme défini dans la revendication 1;

$R_9$ représente un hydrogène, un cyano ou un alcoxycarbonyle en $C_2$—$C_9$; et

Y représente un groupe labile; et, lorsque $R_9$ est un cyano, la conversion du radical cyano en un radical carboxy, et, au besoin, l'élimination ultérieure du ou des radicaux amino-protecteurs.

13. Un procédé de préparation d'un composé comme défini dans la revendication 1 où m est zéro, $R_2$ est un méthyle et $R_c$ est un carboxy qui comprend l'hydrolyse acide d'un dérivé diphtalimido d'un composé répondant à la formule générale XII suivante:

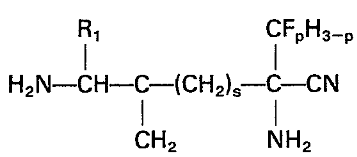

Formule XII

dans laquelle:

$R_1$ et p sont comme défini dans la revendication 1; et s est 1 ou 2.